# DEMANDE DE BREVET EUROPEEN

(11) **EP 4 197 691 A1**
(43) Date de publication de la demande: **21.06.2023**
(21) Numéro de dépôt: 22213983.4
(22) Date de dépôt: 15.12.2022
(51) Int. Cl.: B23K 35/30, C04B 37/02, C22C 5/02

(54) **PROCÉDÉ D'ASSEMBLAGE D'UNE PIÈCE EN ZIRCONE AVEC UN ÉLÉMENT EN TITANE**

(30) Priorité: 17.12.2021 FR 2113878
(71) Demandeur: Commissariat à l'énergie atomique et aux énergies alternatives, 75015 Paris (FR); Oticon Medical A/S, 2765 Smørum (DK); Institut Polytechnique de Grenoble, 38031 Grenoble Cedex 1 (FR); UNIVERSITÉ GRENOBLE ALPES, 38400 Saint-Martin-d'Heres (FR); CENTRE NATIONAL DE LA RECHERCHE SCIENTIFIQUE, 75794 Paris Cedex 16 (FR)
(72) Inventeur: FISCHER, Marie, 38054 GRENOBLE Cedex 09 (FR); CHAUMAT, Valérie, 38054 GRENOBLE CEDEX 09 (FR); GODINOT, Camille, 38054 GRENOBLE Cedex 09 (FR); HODAJ, Fiqiri, 38100 GRENOBLE (FR); MERLET, Ania, 83440 FAYENCE (FR); TOURREL, Guillaume, 06640 SAINT-JEANNET (FR)
(74) Mandataire: Brevalex

(57) **Abrégé**

Procédé d'assemblage d'une pièce (20) en zircone avec un élément (10) en titane avec une brasure, le procédé comprenant les étapes suivantes :
- revêtir une surface de l'élément en titane (10) par une couche (30) de niobium,
- positionner une brasure (40) entre la pièce (20) en zircone et la couche (30) de niobium, la brasure (40) étant en or ou en un alliage d'or,
- chauffer l'ensemble à une température supérieure à la température de fusion de la brasure (40), puis refroidir l'ensemble, moyennant quoi on obtient un assemblage comprenant la pièce en zircone (20) et l'élément en titane (10) assemblés par un joint de brasure comprenant une première partie (40) en or ou en un alliage d'or, une deuxième partie formée d'une couche réactionnelle (35) comprenant des intermétalliques du système AuNbTi, et une troisième partie formée d'une couche réactionnelle d'oxyde.

## Description

### DOMAINE TECHNIQUE

La présente invention se rapporte au domaine général des procédés d'assemblage par brasage de pièces en céramique avec des éléments métalliques.

L'invention concerne un procédé d'assemblage par brasage d'une pièce en zircone avec un élément en titane.

L'invention concerne également un assemblage obtenu par un tel procédé.

L'invention trouve des applications dans de nombreux domaines industriels, et notamment dans le domaine biomédical, par exemple pour la fabrication d'implants auditifs, notamment des implants cochléaires, ou de stimulateurs cardiaques, ou d'implants orthopédiques ou dentaires, ou encore dans le domaine de l'horlogerie.

L'invention est particulièrement intéressante puisqu'elle permet de créer des interfaces fortes lors de l'assemblage et ainsi d'obtenir des pièces présentant de très bonnes propriétés mécaniques.

### ÉTAT DE LA TECHNIQUE ANTÉRIEURE

Le brasage est une technique d'assemblage qui consiste à positionner un matériau d'apport, appelé brasure ou alliage de brasage, entre les pièces à assembler ou à proximité, puis à le faire fondre afin qu'il remplisse le jeu entre les pièces. Après, refroidissement, il forme une liaison entre les pièces à braser. Les pièces sont ainsi assemblées par un joint de brasure. Le matériau d'apport présente une température de fusion inférieure à celles des matériaux à braser, ainsi il n'y a pas fusion des pièces à assembler lors du procédé de brasage. Pour obtenir un bon brasage, il est nécessaire d'avoir un bon mouillage des surfaces à braser par la brasure liquide et la formation de liaisons fortes aux interfaces brasure / pièces à braser.

Depuis les années 2000, le brasage zircone / métal et notamment le brasage zircone / titane a été largement étudié dans la littérature pour diverses applications.

Cependant, il est connu que la zircone n'est pas mouillable par les métaux liquides Au, Ag, Cu, Ni.

Afin de remédier à cet inconvénient, une solution consiste à utiliser une brasure formée d'une matrice constituée d'un de ces métaux et d'un élément actif tel que Ti ou Zr. L'élément actif permet le mouillage de la zircone. Les brasures largement utilisées sont des alliages Ag-Cu-Ti.

Une autre solution consiste, dans un premier temps, à déposer l'élément actif sur la zircone (PVD, pulvérisation...), puis à réaliser le brasage avec une brasure ne contenant pas d'élément actif (par exemple AgCu). Cependant, ces brasures de type Ag-Cu ne sont pas biocompatibles, de même que les brasures actives constituées de cette matrice Ag-Cu et d'un élément actif (brasures Ag-Cu-Ti).

Autrement, lorsque l'élément métallique à assembler à la zircone est en titane, il est possible de réaliser un brasage zircone/titane en utilisant une brasure ne contenant pas d'élément actif et sans métallisation préalable. En effet, dans ce cas, l'élément actif Ti est fourni par le substrat en titane par dissolution et diffusion de Ti à travers la brasure. Celle-ci s'enrichit en élément titane qui va réagir avec la zircone en formant un produit de réaction mouillable (oxyde de titane). Ainsi cela permet le mouillage de la céramique et également l'adhérence de la brasure à la zircone.

Afin d'avoir une brasure biocompatible, il est possible de choisir une brasure en or [1-5] pour le brasage de la zircone avec le titane. Les brasages décrits peuvent être réalisés sans métallisation de la zircone [1-4] ou avec métallisation de la zircone [5].

Par exemple, dans les références [1-2], le brasage de la zircone et du titane est réalisé avec une feuille d'or de 25 µm d'épaisseur sous vide à 1065-1100°C. La formation d'un oxyde de titane TiₓO_{y} à l'interface avec la zircone et des intermétalliques du système Ti-Au côté Ti sont observés.

L'effet de la température de brasage et de la durée du palier de brasage sur les interfaces et la microstructure dans des joints ZrO_{2/}Au/Ti, obtenus avec des feuilles d'or de 50 µm d'épaisseur sous vide (1.3×10⁻³ Pa) à 1110-1190°C pendant 5 - 30 min a également été étudié [3]. La microstructure détectée côté Ti est constituée de 4 composés intermétalliques du système Ti-Au, dont l'épaisseur globale varie avec la durée du maintien et la température (environ 34µm pour 10 min à 1150°C). Côté zircone, une fine couche réactionnelle d'oxyde de titane TiₓO_{y} est détectée et son épaisseur varie avec le temps et la température. Une contrainte optimale de cisaillement de 35 MPa a été obtenue sur une jonction brasée à 1150°C pendant 10 min.

Enfin, Fischer et al.[4] ont étudié le mouillage de la zircone par des alliages Au-Ti et le brasage ZrO₂/Au/Ti. L'or liquide ne mouille pas la zircone. L'addition de quelques pourcents de Ti dans l'or permet le mouillage de la zircone, grâce à la formation d'une couche de réaction TiₓO_{y} à l'interface Au/zircone. L'angle de contact (θ) des alliages Au-Ti sur la zircone décroît quand la teneur en Ti dans l'or croît, jusqu'à atteindre θ = 44±3° à 1150°C avec 3% en masse de Ti dans Au. Le brasage de la zircone au titane avec l'or pur a été réalisé avec succès à 1100°C pendant 15 min, grâce à l'élément actif Ti, provenant du substrat Ti. Dans la configuration d'assemblage ZrO₂/Au/Ti, l'élément actif (Ti) dans l'or liquide est fourni d'abord par dissolution du substrat en titane et ensuite par diffusion de Ti à travers les différentes couches de composés intermétalliques Au-Ti formés à l'interface Ti/Au. En parallèle, le titane diffuse à travers l'or liquide vers l'interface ZrO₂/Au où il réagit avec la zircone et forme un produit de réaction TiₓO_{y} mouillable, ce qui conduit à un bon mouillage de la zircone par l'or liquide.

La tenue mécanique des joints formés par brasage direct ZrO₂/Au/Ti reste néanmoins insuffisante.

Dans le document [5], le procédé de brasage zircone/métal est réalisé avec une brasure d'or. La zircone est préalablement métallisée par une première couche de métallisation en titane, puis par une seconde couche de métallisation en niobium. La couche de titane assure l'accroche à la zircone, et permet aussi l'accroche de la seconde couche de niobium. Cette dernière permettrait l'accroche de la brasure d'or qui adhère à l'élément métallique (en titane par exemple).

Cependant, dans ce procédé, afin de favoriser l'accroche de la première couche de métallisation en titane sur la zircone, l'état de la surface de la céramique doit être altéré pour augmenter sa rugosité de surface, par exemple par une technique de sablage. De plus, ce procédé nécessite un traitement thermique de libération de contrainte réalisé sous charge sur le substrat métallique avant brasage.

Ces différentes étapes (altération de la surface de la zircone et traitement thermique de libération de contrainte) et la nécessité d'une double métallisation complexifient le procédé.

### EXPOSÉ DE L'INVENTION

Un but de la présente invention est de proposer un procédé d'assemblage d'une pièce en zircone remédiant aux inconvénients de l'art antérieur et permettant d'obtenir un assemblage présentant une bonne tenue mécanique.

Pour cela, la présente invention propose un procédé d'assemblage d'une pièce en zircone avec une pièce en titane avec une brasure, le procédé comprenant les étapes suivantes :
- de préférence, nettoyer la pièce en zircone et/ou l'élément en titane et/ou la brasure, par exemple il peut s'agir d'une étape au cours de laquelle le titane est préalablement décapé chimiquement,
- revêtir une surface à braser de l'élément en titane par une couche de niobium,
- positionner une brasure entre la pièce en zircone et la couche de niobium, la brasure étant en or ou en un alliage d'or.
- chauffer l'ensemble à une température supérieure à la température de fusion de la brasure, puis refroidir l'ensemble, moyennant quoi on obtient un assemblage comprenant la pièce en zircone et l'élément en titane assemblés par un joint de brasure comprenant une première partie en or ou en un alliage d'or, une deuxième partie formée d'une couche réactionnelle comprenant des intermétalliques du système AuNbTi, et une troisième partie formée d'une couche réactionnelle d'oxyde.

La couche réactionnelle comprenant des intermétalliques du système AuNbTi est positionnée entre la première partie en or ou en alliage d'or et l'élément en titane. Elle résulte de la réaction de la couche de niobium avec l'or présent dans la brasure et avec le titane de l'élément en titane. Elle comprend voire est constituée d'intermétalliques du système ternaire AuNbTi. Par exemple, elle est formée d'intermétalliques du système ternaire AuNbTi dans le cas d'une brasure d'or pur ou elle est formée principalement d'intermétalliques du système ternaire AuNbTi dans le cas d'une brasure d'alliage d'or.

La première partie peut avoir la même composition que la brasure ou être légèrement enrichie en titane par rapport à la composition initiale de la brasure (typiquement une composition avec 0,1 à 5% en masse de titane, de préférence 0,1 à 2% en masse de titane).

De plus, à l'issue du procédé, une couche réactionnelle d'oxyde est présente entre la première partie en or ou en alliage d'or du joint de brasure et la pièce en zircone. Il s'agit d'une fine couche réactionnelle d'oxyde. L'épaisseur de cette couche d'oxyde est faible (généralement inférieure à 2 µm voire submicronique, c'est-à-dire inférieure à µm). De préférence, la couche d'oxyde a une épaisseur comprise entre 100 nm et 300 nm.

La présence de cette couche permet le mouillage et l'adhérence entre l'or et la zircone lors du brasage.

A titre illustratif et non limitatif, la couche d'oxyde peut être :
- une couche d'oxyde de titane TiₓO_{y},
- une couche comprenant un mélange de plusieurs oxydes, et notamment un mélange des oxydes de titane et du ou des métaux entrant dans la composition de l'alliage d'or (par exemple un mélange des oxydes de titane et de zirconium dans le cas de la brasure AuxZr), ou
- une couche d'un oxyde mixte de titane et du ou des métaux entrant dans la composition de l'alliage d'or (par exemple un oxyde mixte de titane et de zirconium dans le cas de la brasure AuxZr).

Par pièce en zircone, on entend ici et par la suite, une pièce en oxyde de zirconium (ZrO₂) ou une pièce à base d'oxyde de zirconium. De préférence, la pièce à base d'oxyde de zirconium est une pièce en zircone yttriée. La zircone yttriée peut par exemple être une zircone Y-TZP ('Yttrium-Tetragonal Zirconia Polycrystal') stabilisée à 3% molaire d'Y₂O₃. Enfin, la pièce à base d'oxyde de zirconium peut être une pièce en un matériau composite alumine-zircone, tels que les ZTA ('Zirconia Toughened Alumina') à matrice alumine et les ATZ ('Alumina Toughened Zirconia') à matrice zircone. Ces composites allient les propriétés des zircones et des alumines.

L'invention se distingue fondamentalement de l'art antérieur, d'une part, par le dépôt d'une couche de niobium sur la pièce en titane pour la métalliser, et d'autre part, par l'absence de métallisation de la pièce en zircone.

Cette couche de métallisation sur l'élément en titane joue un rôle de modificateur dans la formation de l'interface Ti/Au. En effet, lors du procédé de brasage, la présence de niobium modifie l'interface Ti/Au, c'est à dire la morphologie, l'épaisseur et la nature des intermétalliques formés à cette interface. Les faciès de rupture, après essais mécaniques, sont par ailleurs modifiés par rapport à ceux obtenus sur des assemblages brasés sans métallisation du titane.

L'assemblage obtenu par brasage avec le dépôt de la couche de Nb sur le titane présente une très bonne tenue mécanique.

En particulier, les assemblages obtenus avec un tel procédé présentent, lors des essais de traction, des valeurs de contraintes à rupture :
- nettement supérieures à celles obtenues par brasage direct zircone/Au/titane sans métallisation préalable du titane, et
- très nettement supérieures à celles d'un brasage d'une zircone métallisée par une bicouche Ti+Nb avec un élément en titane.

Selon une variante avantageuse, la brasure est en or.

Selon une autre variante avantageuse, la brasure d'or est un alliage d'or comprenant entre 96 et 99,5% massique d'or.

Avantageusement, la brasure est un alliage d'or, l'alliage d'or comprenant de l'or, du titane ou du zirconium et éventuellement du nickel. Par exemple, l'alliage d'or comprend de l'or, du titane et du nickel.

Selon cette variante avantageuse, la brasure comprend entre 0,5 et 4% massique de titane ou de zirconium et éventuellement de nickel.

De préférence, la brasure comprend de 0,5% à 3% massique de titane.

Avantageusement, la brasure est une brasure de composition Au3Ni0,6Ti (% massique), AuxTi ou AuxZr avec x compris entre 0,5 et 3% massique. Par exemple, il peut s'agir de 0,5% Ti - 99,5 % Au.

Avantageusement, la brasure est une feuille de brasure ayant une épaisseur comprise entre 25µm et 200µm, de préférence entre 50 et 100 µm.

Avantageusement, l'étape d) est réalisée sous vide secondaire ou sous atmosphère protectrice (sous atmosphère réductrice ou sous gaz neutre).

Le procédé est simple à mettre en œuvre car une seule étape de métallisation est suffisante et il n'y a pas besoin d'augmenter la rugosité de surface de la zircone. Le procédé peut être mis en œuvre avec différentes brasures : des brasures d'or pur ou des brasures en alliage d'or.

L'invention concerne également un assemblage obtenu par le procédé précédemment décrit comprenant successivement :
- une pièce en zircone,
- un joint de brasage (ou joint brasé) comprenant une première partie en or ou en un alliage d'or, une deuxième partie formée d'une couche réactionnelle comprenant, voire constituée, d'intermétalliques du système AuNbTi, et une troisième partie formée d'une couche réactionnelle d'oxyde,
- un élément en titane.

Autrement dit, le joint de brasage comprend, et de préférence, est constitué, depuis la pièce en zircone vers l'élément en titane de : la couche réactionnelle d'oxyde, la partie en or ou en alliage d'or et la couche réactionnelle d'intermétalliques du système AuNbTi.

De préférence, le joint de brasage comprend une première partie en or ou en alliage d'or AuNiTi, AuTi ou AuTiZr et une deuxième partie formée d'une couche réactionnelle comprenant voire constituée d'intermétalliques du système AuNbTi côté titane. Le joint de brasage comprend en outre une troisième partie formée d'une couche réactionnelle d'oxyde (oxyde de titane TiₓO_{y} ou mélange d'oxydes dont l'oxyde de titane, ou oxyde mixte de titane) côté zircone.

Selon une variante de réalisation avantageuse, le joint de brasage est obtenu à partir d'une brasure d'or. Le joint de brasage comprend, d'une part, de l'or et, d'autre part, une couche réactionnelle formée d'intermétalliques du système AuNbTi côté titane. La partie en or du joint de brasage peut être légèrement enrichie en titane. Le titane provient du substrat en titane. Le joint de brasage comprend en outre une couche d'oxyde de titane TiₓO_{y} côté zircone.

Selon une autre variante de réalisation avantageuse, le joint de brasage est obtenu à partir d'une brasure en un alliage d'or de composition Au3NiO,6Ti (% massique). Le joint de brasage comprend d'une part un alliage d'or (AuNiTi) et d'autre part une couche réactionnelle formée principalement d'intermétalliques du système ternaire AuNbTi côté titane. Le joint de brasage comprend en outre une couche d'oxyde de titane TiₓO_{y} côté zircone.

Selon une autre variante de réalisation avantageuse, le joint de brasage est obtenu à partir d'une brasure en un alliage d'or de composition AuxTi ou AuxZr (avec x compris entre 0,5 et 3% massique). Le joint de brasage comprend d'une part un alliage d'or (AuTi ou AuTiZr) et d'autre part une couche réactionnelle formée principalement d'intermétalliques du système ternaire AuNbTi côté titane. Le joint de brasage comprend en outre une couche réactionnelle d'oxyde (oxyde de titane TiₓO_{y} dans le cas AuxTi; oxyde de titane TiₓO_{y} ou mélange d'oxydes dont l'oxyde de titane, ou oxyde mixte de titane dans le cas AuxZr), côté zircone.

Dans ces différentes variantes de réalisation, la couche d'oxyde est une fine couche d'oxyde. De préférence, elle a une épaisseur comprise entre 100 nm et 300 nm.

Avantageusement, la zircone est une zircone yttriée ou un composite alumine-zircone.

Les assemblages obtenus avec ce procédé présentent une très bonne tenue mécanique, notamment de 115MPa à 250MPa en traction.

La présente invention permet ainsi de former un assemblage zircone / titane, avec un bon remplissage du joint par la brasure, des interfaces très fortes et une bonne tenue mécanique. Cet assemblage est biocompatible dans le cas des brasures Au, AuxTi et AuxZr.

L'invention concerne également un implant cochléaire comprenant un assemblage tel que défini précédemment, le joint de brasage de l'assemblage comprenant une première partie en or ou en un alliage d'or AuTi ou AuTiZr, une deuxième partie formée d'une couche réactionnelle comprenant voire constituée d'intermétalliques du système AuNbTi, et une troisième partie formée d'une couche réactionnelle d'oxyde.

D'autres caractéristiques et avantages de l'invention ressortiront du complément de description qui suit.

Il va de soi que ce complément de description n'est donné qu'à titre d'illustration de l'objet de l'invention et ne doit en aucun cas être interprété comme une limitation de cet objet.

### BRÈVE DESCRIPTION DES DESSINS

La présente invention sera mieux comprise à la lecture de la description d'exemples de réalisation donnés à titre purement indicatif et nullement limitatif en faisant référence aux dessins annexés sur lesquels :
Les figures 1A, 1B et 1C représentent de manière schématique et en coupe différentes étapes d'un procédé de fabrication d'un assemblage d'un élément en titane avec une pièce en zircone, selon un mode de réalisation particulier de l'invention.
La figure 2 est un cliché obtenu au microscope électronique à balayage (MEB) d'un assemblage comprenant un élément en titane initialement recouvert par une couche de niobium, un joint de brasure (formé à partir d'une brasure en or) et une pièce en zircone, selon un mode de réalisation particulier de l'invention ; on note la présence de deux zones dans le joint formé : l'or et la couche réactionnelle du système ternaire AuNbTi.
La figure 3 est un cliché obtenu au MEB d'un assemblage comprenant un élément en titane, un joint de brasure (formé à partir d'une brasure en or) et une pièce en zircone, selon un procédé de l'art antérieur ; on note la présence de deux zones dans le joint formé : l'or et la couche réactionnelle avec 4 intermétalliques du système Au-Ti.
La figure 4 représente de manière schématique et en trois dimensions une éprouvette de traction avant brasage comprenant deux pièces en zircone, un élément en titane recouvert de part et d'autre par une couche de niobium, et deux brasures (anneaux en or), selon un mode de réalisation particulier de l'invention, l'insert représente un zoom du centre de l'éprouvette.
La figure 5 est une photographie d'une éprouvette dans un outillage selon un mode de réalisation particulier de l'invention.
Les figure 6A et 6B sont des clichés obtenus au MEB d'un brasage zircone /Au/ titane avec double métallisation de la zircone par Ti+Nb, avant et après traitement thermique respectivement, selon un exemple comparatif.
La figure 7 est un cliché obtenu au MEB d'une interface zircone métallisée Ti+Nb / brasure d'or, après un cycle de brasage, selon un exemple comparatif.

Les différentes parties représentées sur les figures ne sont pas nécessairement représentées à l'échelle, pour rendre les figures plus lisibles.

Les différentes possibilités (variantes et modes de réalisation) doivent être comprises comme n'étant pas exclusives les unes des autres et peuvent se combiner entre elles.

En outre, dans la description ci-après, des termes qui dépendent de l'orientation, comme « sur », d'une structure s'appliquent en considérant que la structure est orientée de la façon illustrée sur les figures.

### EXPOSÉ DÉTAILLÉ DE MODES DE RÉALISATION PARTICULIERS

Bien que cela ne soit aucunement limitatif, l'invention trouve particulièrement des applications dans le domaine biomédical pour la fabrication d'implant auditif, et notamment pour la fabrication d'implant cochléaire, pour la fabrication de stimulateur cardiaque, d'implants orthopédiques ou dentaires, ou encore dans le domaine de l'horlogerie.

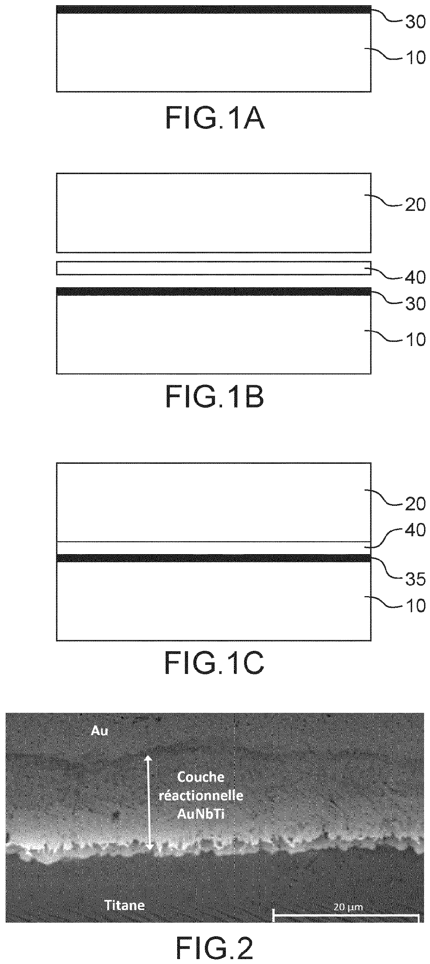

Le procédé d'assemblage d'un élément 10 en titane avec une pièce 20 en zircone avec une brasure comprend les étapes suivantes :
a) de préférence, nettoyer l'élément 10 en titane et/ou la pièce 20 en zircone, ainsi que la brasure 40,
b) recouvrir une surface de l'élément 10 en titane par une couche 30 de niobium (fig. 1A), le titane étant éventuellement préalablement décapé chimiquement,
c) positionner une brasure 40 entre la pièce 20 en zircone et la couche 30 de niobium, la brasure 40 étant en or ou en un alliage d'or (fig. 1B),
d) chauffer l'ensemble à une température supérieure à la température de fusion de la brasure 40, puis refroidir l'ensemble, moyennant quoi on obtient un assemblage comprenant la pièce 20 en zircone et l'élément 10 en titane assemblés par un joint de brasure comprenant une première partie en or ou en un alliage d'or, une deuxième partie formée d'une couche réactionnelle 35 comprenant, voire constituée, d'intermétalliques du système ternaire AuNbTi côté titane (fig. 1C). Une couche réactionnelle d'oxyde (troisième partie) est également présente dans le joint côté zircone. Elle est détectable au microscope électronique en transmission et, selon l'épaisseur et/ou la nature des oxydes, elle peut également être détectée au microscope électronique à balayage (MEB).

La pièce 20 en zircone est en oxyde de zirconium (ZrO₂), ou en zircone yttriée (ZrO₂-Y₂O₃) ou en composite alumine-zircone, tels que les ZTA (Zirconia Toughened Alumina) à matrice alumine et les ATZ (Alumina Toughened Zirconia) à matrice zircone.

La zircone yttriée contient, de préférence, entre 1 et 6 % molaire de Y₂O₃. La zircone yttriée est, par exemple, une zircone Y-TZP ('Yttrium-Tetragonal Zirconia Polycrystal') stabilisée à 3% molaire d'Y₂O₃.

Par « entre X et Y », on entend que les bornes X et Y sont incluses.

Par la suite, nous emploierons le terme zircone, mais il est évident que le terme zircone peut être remplacé par zircone yttriée ou composite alumine-zircone.

Par exemple, pour l'étape a), il est préférable de nettoyer la pièce 20 à braser en zircone, l'élément 10 en titane et la brasure 40 dans un bain d'acétone, puis d'éthanol aux ultrasons.

La surface de l'élément 10 en titane, destinée à être métallisée, peut être soumise à une étape décapage chimique et/ou à une étape un décapage ionique.

De plus, la zircone peut être traitée thermiquement à 1350°C, de préférence sous air (opération dite de grillage), avant l'étape c).

Lors de l'étape b), l'élément 10 en titane à braser est au préalable revêtu d'un dépôt de niobium. Cette couche de métallisation 30 permet de renforcer l'interface Ti/Au par modification, lors du procédé de brasage, de la morphologie, de l'épaisseur, et de la nature des intermétalliques dans le système Ti-Au.

Le dépôt de la couche 30 de niobium est, par exemple, réalisé par dépôt physique en phase vapeur (ou PVD pour 'physical vapor deposition') ou par pulvérisation ('sputtering'). L'épaisseur de niobium déposée va, par exemple, de quelques microns à 20 µm, typiquement 5 à 10 µm.

La pièce 20 en zircone n'est pas métallisée, c'est-à-dire que la brasure 40 est directement en contact avec la surface de la pièce 20 en zircone à assembler.

Il est évident qu'il est possible d'assembler une ou plusieurs pièces en zircone avec un ou plusieurs éléments en titane. Par exemple, il est possible d'assembler deux pièces en zircone avec une pièce en titane. On met alors en œuvre deux brasures et les deux faces de la pièce en titane destinée à être en contact avec les deux brasures sont métallisées.

La brasure 40 est une brasure riche en or telle que de l'or pur ou un alliage d'or.

Par or pur, on entend de l'or ayant une pureté supérieure à 99% massique et de préférence supérieure à 99,5% massique.

L'alliage d'or est par exemple une brasure de composition Au3Ni0,6Ti (% massique), par exemple commercialisée sous la référence GoldABA ou une brasure AuxTi ou AuxZr avec x compris entre 0,5 et 3% massique.

La température de fusion de la brasure est inférieure à la température de fusion de l'élément en titane.

La brasure 40 est, avantageusement, sous la forme d'une feuille.

L'épaisseur de la feuille de brasure est, par exemple comprise entre 25 et 200 µm, de préférence entre 50 et 150 µm.

La brasure 40 peut également être sous la forme d'une poudre.

Lors de l'étape c) et lors de l'étape d) un outillage pour maintenir la pièce 20 en zircone, l'élément en titane 10 et la brasure 30 peut être utilisé.

Lors de l'opération de brasage (étape d), on assemble la pièce de zircone 20 avec l'élément en titane 10 par fusion de la brasure 40. On chauffe l'ensemble de façon à faire fondre la brasure. La température du palier de brasage dépend de la composition de la brasure. La température du palier de brasage est supérieure à la température de fusion de la brasure 40. Typiquement, la température du palier de brasage est supérieure à 1064°C dans le cas de l'or pur et, de préférence, inférieure à 1120°C. Dans le cas de la brasure commerciale GoldABA, le liquidus et le solidus sont, respectivement, de 1030°C et de 1003°C. La température de brasage est de préférence supérieure à 1030°C et inférieure à 1120°C.

La durée du palier de brasage est par exemple comprise entre 0 et 15 minutes, de préférence entre 30 secondes et 10 minutes.

Après le palier de brasage, l'ensemble est refroidi jusqu'à la température ambiante. La brasure se solidifie au refroidissement et les pièces sont alors liées par la brasure solidifiée.

La métallisation de l'élément 10 en titane par le niobium ne conduit pas à la formation de porosités à l'interface titane métallisé Nb/ brasure Au lors du chauffage associé au cycle de brasage, contrairement au cas de la métallisation de la zircone (interface zircone métallisée Ti+Nb / Au fragilisée par la formation de porosités lors du chauffage).

Ce procédé de brasage peut être réalisé sous vide secondaire ou sous atmosphère protectrice (gaz neutre ou atmosphère réductrice). Il est réalisé de préférence dans un four sous vide secondaire.

L'assemblage obtenu par le procédé précédemment décrit comprend successivement :
- une pièce 20 en zircone,
- un joint de brasage comprenant une première partie 40 en or ou en un alliage d'or (du côté de la pièce en zircone 20), une deuxième partie formée d'une couche réactionnelle 35 comprenant, voire constituée, d'intermétalliques du système ternaire AuNbTi côté titane (couche formée par réaction de la couche de métallisation 30 avec la brasure 40 et avec l'élément en titane 10), et, enfin, une troisième partie (non représentée) formée d'une couche d'oxyde côté zircone, et
- un élément en titane 10.

La première partie a la même composition que la brasure ou une composition correspondant à la composition de la brasure légèrement enrichie en titane. La teneur en titane est comprise entre 0,1 % et 5 % en masse de titane, de préférence entre 0,1 et 2 % en Ti.

La couche réactionnelle 35 d'intermétalliques est positionnée entre la première partie 40 en or ou en alliage d'or et l'élément en titane. La couche réactionnelle 35 d'intermétalliques a, par exemple, une épaisseur de 5 µm à 30µm.

La fine couche d'oxyde est positionnée entre la première partie 40 en or ou en alliage d'or et la pièce en zircone. La fine couche d'oxyde a, par exemple, une épaisseur entre 100 nm et 2 µm.

Avantageusement, le joint de brasage comprend un alliage d'or AuNiTi, AuTi ou AuTiZr, une couche réactionnelle 35 comprenant voire constituée d'intermétalliques du système ternaire AuNbTi, et éventuellement une fine couche d'oxyde côté zircone.

Selon une variante de réalisation avantageuse, la zircone est une zircone yttriée.

Selon une autre variante de réalisation avantageuse, la zircone est un composite alumine-zircone.

L'invention est particulièrement intéressante pour former des implants auditifs.

En particulier, on pourra fabriquer un implant cochléaire comprenant un assemblage tel que défini précédemment, le joint de brasage de l'assemblage comprenant, d'une part, de l'or ou un alliage d'or AuTi ou AuTiZr et, d'autre part, une couche réactionnelle 35 comprenant voire constituée d'intermétalliques du système ternaire AuNbTi. Une fine couche réactionnelle d'oxyde est également présente côté zircone.

### Exemples illustratifs et non limitatifs d'un mode de réalisation :

Nous allons maintenant décrire plusieurs exemples non limitatifs de réalisation ainsi que des tests comparatifs.

Dans les exemples et tests suivants, la zircone est de la zircone yttriée à 3% molaire.

### Exemple 1 : Réalisation d'assemblages par brasage zircone /Au/ titane avec des dépôts de Nb d'épaisseur X µm (X=5, 10, 15 µm) sur le titane.

Trois assemblages "disque en zircone 20 (φ15 mm, h 2mm) / disque en titane 10 (φ15 mm, h 2mm)" ont été réalisés par brasage avec une feuille 40 d'or pur. Les disques en titane sont préalablement métallisés par une couche 30 de niobium. Trois épaisseurs différentes ont été testées : 5 µm, 10 µm et 15 µm.

La feuille 40 d'or est découpée de façon à former un disque de diamètre 15 mm et d'épaisseur 100 µm.

Les étapes suivantes ont été réalisées pour faire ces assemblages :
Etape 1 : Nettoyer les pièces à braser en zircone, titane et la brasure d'or dans un bain d'acétone, puis d'éthanol aux ultrasons, le titane étant préalablement décapé chimiquement.
Etape 2 : Métalliser les surfaces à braser des trois pièces en titane en réalisant un décapage ionique du titane suivi d'un dépôt de niobium par PVD. L'épaisseur de niobium est respectivement 5 µm pour l'assemblage N°1, 10 µm pour l'assemblage n°2 et 15 µm pour l'assemblage N°3.
Etape 3 : Déposer la feuille de brasure d'or pur entre les pièces à assembler en zircone et titane. La face du titane revêtue de niobium est celle positionnée vers la brasure.
Etape 4 : Placer les trois ensembles formés des pièces et des brasures, dans un four de brasage sous vide secondaire. Sur chaque ensemble a été placée une petite masse en W de 23 g pour maintenir l'ensemble dans le four.
Etape 5 : Chauffer l'ensemble à 1080°C pendant quelques minutes.
Etape 6 : Après le palier de brasage, l'ensemble est refroidi jusqu'à température ambiante et les pièces sont alors liées par la brasure solidifiée.

Les pièces ont été découpées, enrobées dans une résine époxy et polies. Les joints sont bien remplis par la brasure, il n'y a pas de manque de brasure, c'est-à-dire qu'il n'y a pas de trou dans la brasure. Les interfaces ne sont pas fissurées.

On peut noter que l'interface titane /or (figure 2) n'est pas constituée de couches successives d'intermétalliques Ti-Au contrairement au cas du brasage direct sans métallisation du titane par le niobium.

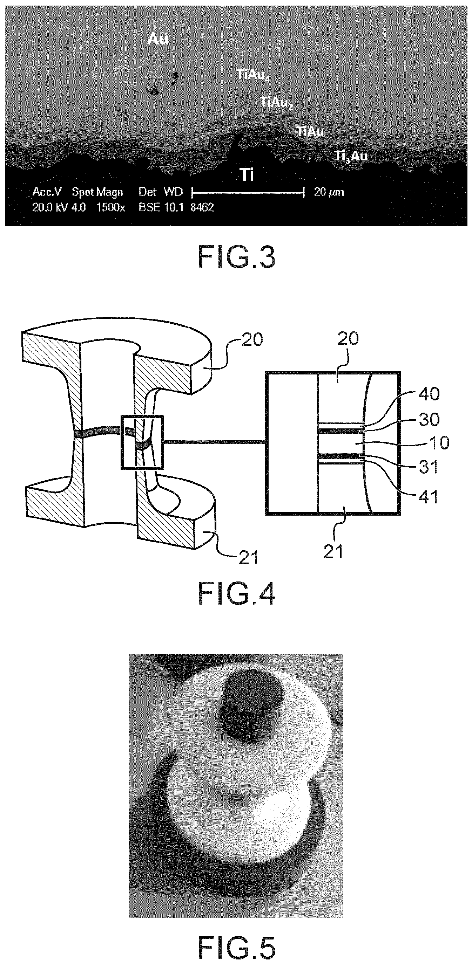

En effet, à titre de comparaison, un assemblage avec un brasage direct zircone/Au/titane sans métallisation du titane par le niobium a été fabriqué puis caractérisé. On observe la présence de 4 couches parallèles et successives d'intermétalliques Ti₃Au, TiAu, TiAu₂, TiAu₄ (figure 3).

### Exemple 2: Fabrication d'éprouvettes de traction par brasage zircone /Au/ titane avec un dépôt de Nb d'épaisseur 5 µm, et tests mécaniques.

Des éprouvettes de traction ont été brasées en se basant sur la norme ASME F19-64 utilisée pour la caractérisation des assemblages céramique/métal. Un schéma présente ce type d'éprouvette dans le cas du brasage zircone / Au / titane (figure 4). Sur ce schéma, on distingue au centre de la pièce l'anneau de titane 10. De part et d'autre de l'anneau sont placés des anneaux de brasure d'or 40, 41, et enfin les pièces 20, 21 en zircone. Chaque côté de l'anneau de titane 10 est recouvert par une couche de niobium 30, 31. L'ensemble est maintenu dans un outillage lors de l'opération de brasage afin qu'il n'y ait pas de désalignement entre les pièces (figure 5).

Le brasage a été réalisé de la façon suivante :
Etape 1 : Nettoyer les pièces à braser en zircone, titane et la brasure d'or dans un bain d'acétone, puis d'éthanol aux ultrasons, le titane étant préalablement décapé chimiquement.
Etape 2 : Métalliser les 2 surfaces à braser des anneaux en titane en réalisant un décapage ionique du titane suivi d'un dépôt de niobium par PVD. L'épaisseur de niobium est de 5 µm.
Etape 3 : Déposer 2 anneaux de brasure d'or pur entre les pièces à assembler en zircone et titane (zircone / Au / titane / Au / zircone).
Etape 4 : Placer chaque éprouvette à braser dans un outillage de maintien et installer l'ensemble dans un four de brasage sous vide secondaire.
Etape 5 : Chauffer l'ensemble à 1080°C pendant quelques minutes.
Etape 6 : Après le palier de brasage, l'ensemble est refroidi jusqu'à température ambiante et les éprouvettes sont alors liées par la brasure solidifiée.

Nous avons ainsi fabriqué 4 éprouvettes. Elles ont été soumises à des essais de traction. Les valeurs de contraintes à rupture sont mesurées. Une contrainte à rupture moyenne de 179 MPa a été obtenue.

### Exemple 3 comparatif : Réalisation d'assemblages zircone/titane et éprouvettes de traction par brasage zircone /Au/ titane sans aucune métallisation des substrats à braser, et tests mécaniques.

Des éprouvettes de traction ont été brasées selon le même protocole que pour l'exemple 2, mais sans faire de métallisation du titane, ni de métallisation de la zircone. Ces éprouvettes sont soumises à l'essai de traction et les valeurs de contraintes à rupture sont mesurées. On obtient après un brasage à 1080°C et quelques minutes de palier, une contrainte à rupture moyenne de 97 MPa, ce qui est nettement inférieur à la contrainte obtenue avec une métallisation du titane par du niobium.

La rupture a lieu entre les intermétalliques TiAu₂ et TiAu₄.

### Exemple 4 comparatif : Réalisation d'assemblages zircone/titane et éprouvettes de traction par brasage zircone /Au/ titane avec double métallisation de la zircone par Ti+Nb, tests mécaniques + Effet de la température seule sur le dépôt

Des éprouvettes de traction ont été brasées selon le même protocole que pour l'exemple 2, mais en réalisant une double métallisation de la zircone par le titane puis le niobium. En revanche l'anneau de titane n'est pas métallisé. Ces éprouvettes sont soumises à l'essai de traction et les valeurs de contraintes à rupture sont mesurées. Pour 6 éprouvettes, on obtient une contrainte à rupture moyenne de 42 MPa, ce qui est significativement inférieur aux contraintes à rupture obtenues avec les éprouvettes de l'exemple 2.

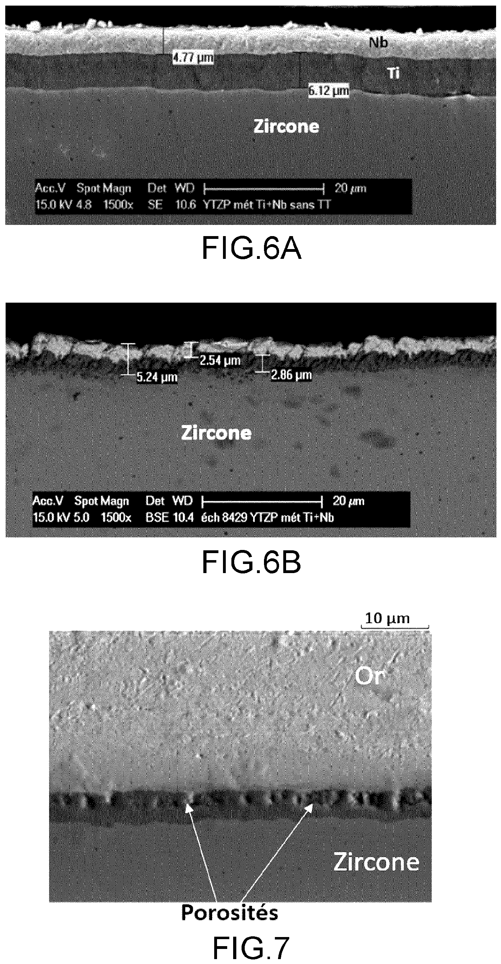

Pour comprendre ce résultat, un traitement thermique a été appliqué, identique à celui du cycle de brasage, sur la zircone métallisée Ti+Nb (sans faire de brasage). Ce traitement conduit à une dégradation du dépôt avec formation de porosités, ce qui affaiblit l'interface côté zircone. Les clichés obtenus au microscope électronique à balayage avant et après traitement thermique sont représentés sur les figures 6A et 6B respectivement.

Après brasage, on retrouve ainsi ces porosités au niveau de l'interface côté zircone (figure 7), confirmant que cette interface représente le point faible de l'assemblage, c'est-à-dire qu'il s'agit du lieu de rupture lors des essais mécaniques.

La dégradation de la métallisation (présence de porosités) lors du cycle de brasage a lieu avant même la fusion de la brasure d'or. Cela conduit à de moins bonnes interfaces qu'un brasage sans métallisation de la zircone. En effet, il semble que c'est la zircone elle-même qui libère de l'oxygène qui vient dégrader la métallisation, cette dernière devenant ainsi le point faible de l'assemblage.

Ceci montre que la métallisation de la zircone ne permet pas d'obtenir un assemblage ayant une bonne tenue mécanique.

### REFERENCES

1 Agathopoulos, S. et al., 'Interactions at Zirconia-Au-Ti Interfaces at High Temperatures'. Key Eng. Mater. 206-213, 487-490 (2001).
2 Agathopoulos, S. et al., 'A review of recent investigations on zirconia joining for biomedical appications'. Key Eng. Mater. 206-213, 487-490 (2001).
3 Lei, Y. et al., 'Evaluation of Biomedical Ti/ZrO2 Joint Brazed with Pure Au Filler: Microstructure and Mechanical Properties'. Metals 10, 526 (2020).
4 Fischer M. et al., 'Wetting and reactivity of zirconia by Au-Ti active alloys in view of zirconia / titanium brazing with pure gold'. Proceedings de la conférence internationale IBSC 2021 (International Brazing and Soldering Conference. 3-6 Octobre 2021).
5 FR3051131-A1 'Procédé de brasage d'un élément métallique sur une pièce de zircone et dispositif implantable brasé'

## Revendications

1. Procédé d'assemblage d'une pièce (20) en zircone avec un élément (10) en titane avec une brasure, le procédé comprenant les étapes suivantes :
- de préférence, nettoyer la pièce (20) en zircone et/ou l'élément (10) en titane et/ou la brasure (40),
- revêtir une surface de l'élément en titane (10) par une couche (30) de niobium,
- positionner une brasure (40) entre la pièce (20) en zircone et la couche (30) de niobium, la brasure (40) étant en or ou en un alliage d'or,
- chauffer l'ensemble à une température supérieure à la température de fusion de la brasure (40), puis refroidir l'ensemble, moyennant quoi on obtient un assemblage comprenant la pièce en zircone (20) et l'élément en titane (10) assemblés par un joint de brasure comprenant une première partie (40) en or ou en un alliage d'or, une deuxième partie formée d'une couche réactionnelle (35) comprenant des intermétalliques du système AuNbTi, et une troisième partie formée d'une couche réactionnelle d'oxyde.

2. Procédé selon la revendication 1, **caractérisé en ce que** la brasure (40) est en or.

3. Procédé selon la revendication 1, **caractérisé en ce que** la brasure (40) est un alliage d'or, l'alliage d'or comprenant de l'or, du titane ou du zirconium, et éventuellement du nickel.

4. Procédé selon la revendication précédente, **caractérisé en ce que** la brasure comprend entre 0,5 et 4% massique de titane ou de zirconium et éventuellement de nickel.

5. Procédé selon l'une quelconque des revendications 3 et 4, **caractérisé en ce que** la brasure est une brasure de composition Au3Ni0,6Ti, AuxTi ou AuxZr avec x compris entre 0,5 et 3% massique.

6. Procédé selon l'une quelconque des revendications précédentes, **caractérisé en ce que** la brasure (40) est une feuille de brasure ayant une épaisseur comprise entre 25µm et 200µm.

7. Procédé selon l'une quelconque des revendications précédentes, **caractérisé en ce que** l'étape d) est réalisée sous vide secondaire, sous atmosphère réductrice ou sous gaz neutre.

8. Assemblage comprenant successivement :
- une pièce (20) en zircone,
- un joint de brasage comprenant une première partie (40) en or ou en un alliage d'or, une deuxième partie formée d'une couche réactionnelle (35) comprenant des intermétalliques du système AuNbTi, et une troisième partie formée d'une couche réactionnelle d'oxyde,
- un élément en titane (10).

9. Assemblage selon la revendication 8, **caractérisé en ce que** le joint de brasage comprend une première partie (40) en un alliage d'or AuNiTi, AuTi ou AuTiZr, une deuxième partie formée d'une couche réactionnelle (35) comprenant des intermétalliques du système AuNbTi, et une troisième partie formée d'une couche réactionnelle d'oxyde.

10. Assemblage selon l'une des revendications 8 et 9, **caractérisé en ce que** la zircone est une zircone yttriée.

11. Assemblage selon l'une des revendications 8 et 9, **caractérisé en ce que** la zircone est un composite alumine-zircone.

12. Implant cochléaire comprenant un assemblage tel que défini dans l'une des revendications 8 à 11, le joint de brasage de l'assemblage comprenant une première partie (40) en or ou en un alliage d'or AuTi ou AuTiZr, une deuxième partie formée d'une couche réactionnelle (35) comprenant des intermétalliques du système AuNbTi, et une troisième partie formée d'une couche réactionnelle d'oxyde.
